(19) Europäisches Patentamt
European Patent Office
Office européen des brevets

(11) Veröffentlichungsnummer: **0 444 265 B1**

# (12) EUROPÄISCHE PATENTSCHRIFT

(45) Veröffentlichungstag der Patentschrift: **27.07.94**

(51) Int. Cl.⁵: **C07C 6/04**, C07C 67/475, C07C 11/02, C07C 69/533, //B01J31/32

(21) Anmeldenummer: **90123931.9**

(22) Anmeldetag: **12.12.90**

## (54) Verfahren zur Metathese von Olefinen und funktionalisierten Olefinen.

(30) Priorität: **02.03.90 DE 4006539**

(43) Veröffentlichungstag der Anmeldung:
**04.09.91 Patentblatt 91/36**

(45) Bekanntmachung des Hinweises auf die Patenterteilung:
**27.07.94 Patentblatt 94/30**

(84) Benannte Vertragsstaaten:
**AT BE CH DE DK ES FR GB IT LI NL**

(56) Entgegenhaltungen:
**DE-A- 3 229 419**
**FR-A- 2 521 872**

**JOURNAL OF ORGANOMETALLIC CHEMISTRY, Band 255, 1983, Lausanne R.H.A. BOSMA etal. "Heterogenous metathesis of unsaturated esters using a rhenium-basedcatalyst" Seiten 159-171**

**CHEMICAL ABSTRACTS, Band 105, Nr. 17, 27. Oktober 1986, Columbus, Ohio, USA; XU, XIAODING et al.: "Rhenium (VII) oxide/alumina-boron oxide metathesis catalysts", Seite 636, Spalte 2, Zusammenfassung-Nr. 152 416q**

(73) Patentinhaber: **HÜLS AKTIENGESELLSCHAFT**

**D-45764 Marl(DE)**

(72) Erfinder: **Warwel, Siegfried, Prof. Dr.**
**Hans-Böckler-Allee 81**
**W-5100 Aachen(DE)**
Erfinder: **Jägers, Hans-Gerd, Dr.**
**Josefstrasse 79**
**W-4390 Gladbeck(DE)**
Erfinder: **Ercklentz, Barbara**
**Kaesbachstrasse 12**
**W-4050 Mönchengladbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Metathese von Olefinen und funktionalisierten Olefinen an $Re_2O_7/Al_2O_3$-haltigen Katalysatoren, bei dem neue Aktivatoren verwendet werden.

Die Metathese olefinischer Kohlenwasserstoffe dient in der Technik zur Herstellung von speziellen Olefinen, Di- und Polyenen sowie ungesättigten Polymeren. Auch Olefine mit funktionellen Gruppen unterliegen der Metathesereaktion, sofern geeignete Katalysatoren zur Anwendung kommen. Von besonderer Bedeutung ist hierbei die Metathese ungesättigter Fettsäuremethylester, die durch Umesterung nativer Fette und Öle mit Methanol im großindustriellen Maßstab produziert werden und damit als Ausgangsverbindungen wirtschaftlich zur Verfügung stehen. Durch Metathese dieser Ester eröffnen sich neue einfache Zugänge zu chemisch-technisch Zwischenprodukten für die industrielle Herstellung von Tensiden, Kunststoffen, Kunststoffweichmachern, Schmierölen sowie einer ganzen Palette von Feinchemikalien.

Warwel, Erdöl-Erdgas-Kohle 103 (1987), 238 - 45, beschreibt industrielle Metatheseprozesse, bei denen vorwiegend $Re_2O_7/Al_2O_3$-, $CoO$-$MoO_3/Al_2O_3$- und $WO_3/SiO_2$-Katalysatoren verwendet werden. Danach ist nur der $Re_2O_7/Al_2O_3$-Katalyator bereits bei Raumtemperatur aktiv. Als Aktivatoren der Katalysatoren werden dabei im allgemeinen Zinnalkyle eingesetzt.

Nach FR 2 521 872 können bei der Metathese auch Bleitetraalkyle als Aktivatoren eingesetzt werden.

Aluminiumorganische Verbindungen wurden bisher noch nicht als Aktivatoren empfohlen. Bosma et al., Journal of Organometallic Chemistry 255 (1983), 159 - 71, testeten zwar eine Reihe metallorganischer Verbindungen und dabei auch $(CH_3)_3Al_2Cl_3$, stellten jedoch fest, daß von dieser aluminiumorganischen Verbindung nur eine geringe aktivierende Wirkung ausging.

Bei bestimmten Anwendungen der durch Olefin-Metathese hergestellten olefinischen Verbindungen, z. B. im pharmazeutischen oder kosmetischen Sektor, ist der Einsatz von Zinn- und Bleialkylen als Aktivatoren für die Metathesekatalysatoren jedoch aus physiologischen Gründen bedenklich. Es kann nämlich nicht ausgeschlossen werden, daß die metallorganischen Verbindungen oder ihre Folgeprodukte nach Abtrennung des heterogenen Rheniumoxid-Kontakts in geringen Mengen im olefinischen Reaktionsgemisch verbleiben und damit letztlich in das aus den Olefinen hergestellte technische Endprodukt eingeschleppt werden.

Es stellte sich daher die Aufgabe, gute Aktivatoren zu entwickeln, die physiologisch unbedenklich sind.

Die Aufgabe wird erfindungsgemäß dadurch gelöst, daß man halogenfreie Organoaluminiumverbindungen als Aktivatoren einsetzt.

Vorzugsweise verwendet man Organoaluminiumverbindungen der Typen $AlR_3$, $XAlR_2$ und $X_2AlR$. Dabei steht R für Alkyl mit 1 bis 8 Kohlenstoffatomen. X ist ein Alkoxy mit ebenfalls 1 bis 8 Kohlenstoffatomen. Beispiele für den Substituenten R sind Methyl, Ethyl, Propyl, n-Butyl, iso-Butyl und Hexyl. X ist beispielsweise Methoxy, Ethoxy oder n-Butoxy.

Weitere vorzugsweise eingesetzte Organoaluminiumverbindungen sind Alkylaluminoxane der Formel

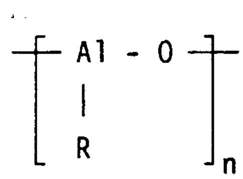

Dabei bedeutet R Alkyl mit 1 bis 8 Kohlenstoffatomen. Beispiele dafür sind Methyl, Ethyl und n-Butyl. Methylaluminoxane werden ganz besonders bevorzugt. Die Zahl n liegt im Bereich von 2 bis 30, wobei eine Zahl von 5 bis 20 stark bevorzugt wird.

Alkylaluminoxane können durch Umsetzung von Aluminiumtrialkyl mit Wasser hergestellt werden.

Bei der Metathese können die Organoaluminiumverbindungen in Substanz oder als Lösung in einem organischen Lösemittel zugesetzt werden. Geeignete Lösemittel sind wasserfreie Ether, Kohlenwasserstoffe und Halogenkohlenwasserstoffe.

Die Mengenverhältnisse von Katalysator zu Aktivator können durch das molare Verhältnis $Re_2O_7$ : Aluminium in der Organoaluminiumverbindung beschrieben werden. Diese Verhältnis liegt vorzugsweise bei 1 : 1 bis 1 : 10.

Bei einer Aktivatormenge unterhalb des Mindestwertes geht der Umsatz bei der Metathese deutlich zurück. Bei einer Aktivatorkonzentration oberhalb der hier genannten Höchstmenge - derartige Konzentrationen können auch eingestellt werden - wird keine weitere Verbesserung mehr erzielt.

Bei der Metathese wird ein $Re_2O_7/Al_2O_3$-haltiger Trägerkatalysator verwendet. Der katalytisch aktive Bestandteil kann dabei alleine aus $Re_2O_7$ bestehen. Neben $Re_2O_7$ können jedoch auch weitere Oxide, wie beispielsweise $B_2O_3$, $MoO_3$, $WO_3$ oder $V_2O_5$, enthalten sein. Der Träger kann aus $Al_2O_3$ bestehen. Neben $Al_2O_3$ können auch weitere, oxidische Bestandteile, wie z. B. $SiO_2$, enthalten sein. Vorzugsweise wird ein $B_2O_3$-$Re_2O_7/Al_2O_3$-$SiO_2$-Katalysator verwendet.

Für die Metathese können lineare, verzweigte und auch cyclische Olefine eingesetzt werden. Diese Verbindungen können im allgemeinen auch ohne Aktivator metathetisiert werden. Ein Aktivator erhöht hier den Umsatz.

Funktionalisierte Olefine im Sinne der Erfindung sind ungesättigte Ester, Ether, Halogen- und Stickstoffverbindungen, Aldehyde, Ketone sowie derivatisierte Alkohole und derivatisierte Carbonsäuren. Vorzugsweise werden ungesättigte Carbonsäureester eingesetzt.

Bei der Metathese von funktionalisierten Olefinen wird im allgemeinen ein Aktivator benötigt, da ohne Aktivator eine Umsetzung nicht erfolgt. Das gilt im besonderen für die Metathese von ungesättigten Estern.

Die Metathesereaktionen können als Homo-Metathese, als Co-Metathese (Einsatz zweier unterschiedlicher olefinischer Verbindungen) und bei Cycloolefinen als Substrat als metathetische, ringöffnende Polymerisation durchgeführt werden. Die Metathesereaktionen werden bevorzugt bei Raumtemperatur durchgeführt, was aus Gründen der Energieeinsparung vorteilhaft ist. Die Anwendung tieferer oder höherer Temperaturen ist jedoch auch möglich.

Das erfindungsgemäße Verfahren zur Metathese von Olefinen und funktionalisierten Olefinen ermöglicht hohe Umsätze und die Bildung neuer Produkte mit hoher Selektivität. Nach Abtrennung des Katalysators sind die Produkte frei von giftigen Blei- und Zinnverbindungen, die bisher als Aktivatoren für die Metathese funktionalisierter Olefine eingesetzt werden. Die Produkte sind deshalb physiologisch unbedenklich.

Die folgenden Beispiele sollen die Erfindung erläutern.

Beispiel 1

Co-Metathese von Ölsäuremethylester mit 4-Octen

Katalysator: $B_2O_3$-$Re_2O_7/Al_2O_3$-$SiO_2$, aktiviert mit Methylaluminoxan

Als Katalysator wird ein $B_2O_3$-$Re_2O_7/Al_2O_3$-$SiO_2$-Trägerkatalysator verwendet. Das Trägermaterial ist ein pulverförmiges, amorphes Alumosilikat mit 40 Gewichtsprozent $SiO_2$. Der Katalysator enthält 5,8 Gewichtsprozent $B_2O_3$ und 4,4 Gewichtsprozent $Re_2O_7$, jeweils bezogen auf den Alumosilikatträger.

Als Aktivator wird eine 9,5%ige Methylaluminoxan-Lösung in Toluol eingesetzt. Formel:

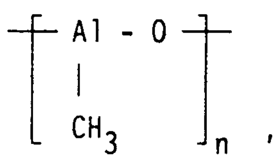

Dabei ist n etwa 15, während das mittlere Molekulargewicht etwa 900 beträgt.

In einem mit $N_2$ gefüllten 50 ml fassenden zylindrischen Schlenkgefäß mit Magnetrührkern werden 0,58 g $B_2O_3$-$Re_2O_7/Al_2O_3$-$SiO_2$-Katalysator (mit 0,053 mmol $Re_2O_7$) vorgelegt. Anschließend werden 0,11 ml Methylaluminoxan-Lösung (0,212 mmol Al) sowie 1 ml n-Heptan zugegeben, wobei sich der Trägerkontakt dunkelbraun färbt.

Anschließend werden 4,1 ml 4-Octen (26 mmol) und 4,25 ml Ölsäuremethylester (13 mmol) zugegeben, wodurch ein molares Verhältnis der einzelnen Komponenten wie folgt gegeben ist: $Re_2O_7$ : Al : Ölsäuremethylester : 4-Octen = 1 : 4 : 250 : 500. Dabei ist Al das Aluminium in der Organoaluminiumverbindung.

Die Mischung wird 2 Stunden bei Raumtemperatur gerührt. Der pulverförmige Trägerkatalysator wird absitzen gelassen. Von der überstehenden Lösung wird eine Probe entnommen, die nach Zugabe einiger Tropfen Methanol zur Zerstörung etwaiger Katalysatorreste gaschromatographisch analysiert wird. Gemäß der Reaktionsgleichung

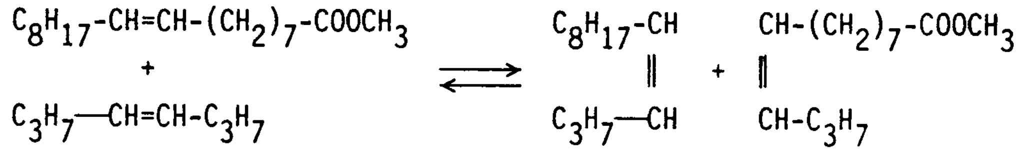

enthält die Reaktionsmischung neben den Einsatzstoffen 4-Tridecen und 9-Tridecensäuremethylester. Der Umsatz des eingesetzten Ölsäuremethylesters beträgt 56 %, die Selektivität zu den Metatheseprodukten 88 %.

Beispiel 2

Der Versuch aus Beispiel 1 wird wiederholt. Dabei wird jedoch etwa die dreifache Katalysator- und Aktivatormenge eingesetzt. Nun ist das molare Verhältnis von $Re_2O_7$ : Al : Ölsäuremethylester : 4-Octen = 1 : 4 : 80 : 160. Man erzielt einen Ölsäuremethylester-Umsatz von 78 % bei einer Selektivität von 89 %.

Beispiel 3
Co-Metathese von 10-Undecensäuremethylester mit 4-Octen
Katalysator: $B_2O_3$-$Re_2O_7$/$Al_2O_3$-$SiO_2$, aktiviert mit Methylaluminoxan

Der Versuch aus Beispiel 1 wird wiederholt, wobei jedoch Ölsäuremethylester durch 10-Undecensäuremethylester ersetzt wird. Gemäß der Reaktionsgleichung

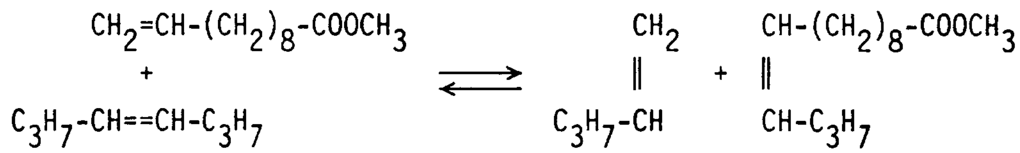

enthält das Reaktionsgemisch neben den Einsatzstoffen 1-Penten und 10-Tetradecensäuremethylester.

Bei einem molaren Verhältnis von $Re_2O_7$ : Al : 10-Undecensäuremethylester = 1 : 4 : 250 : 500 wird ein Umsatz von 61 % bei einer Selektivität von 84 % erreicht.

Beispiel 4

Der Versuch aus Beispiel 3 wird wiederholt, wobei jedoch etwa die dreifache Menge an Katalysator und Aktivator eingesetzt wird. Dabei beträgt das molare Verhältnis von $Re_2O_7$ : Al : 10-Undecensäuremethylester : 4-Octen = 1 : 4 : 80 : 160. Der Umsatz des 10-Undecensäuremethylester steigt auf 81 % bei einer Selektivität zu den Metatheseprodukten von 87 %.

Beispiel 5
Co-Metathese von 10-Undecensäuremethylester mit 4-Octen
Katalysator: $Re_2O_7$/$Al_2O_3$-$SiO_2$, aktiviert mit Methylaluminoxan

Der Versuch aus Beispiel 3 wird wiederholt, jedoch enthält der Rheniumoxid-Trägerkatalysator jetzt kein $B_2O_3$.

Bei einem molaren Verhältnis von $Re_2O_7$ : Al : 10-Undecensäuremethylester : 4-Octen = 1 : 4 : 250 : 500 beträgt der Undecensäure-Umsatz jetzt 44 % bei einer Selektivität von 82 %.

Beispiel 6
Co-Metathese von 10-Undecensäuremethylester mit 4-Octen
Katalysator: $MoO_3$-$Re_2O_7$/$Al_2O_3$, aktiviert mit Methylaluminoxan

Der Versuch aus Beispiel 3 wird wiederholt, jedoch wird jetzt $MoO_3$-$Re_2O_7$ auf $Al_2O_3$ als Rhenium-Trägerkatalysator eingesetzt, der 5 Gewichtsprozent $MoO_3$ und 3,5 Gewichtsprozent $Re_2O_7$, jeweils bezogen auf das Trägermaterial, enthält. Außerdem wird im Vergleich zu Beispiel 3 etwa die fünffache Katalysator- und Aktivatormenge eingesetzt.

Bei einem molaren Verhältnis von $Re_2O_7$ : Al : 10-Undecensäuremethylester : 4-Octen = 1 : 5 : 50 : 100 beträgt der 10-Undecensäuremethylester-Umsatz 20 %.

Beispiel 7
Co-Metathese von 10-Undecensäuremethylester mit 4-Octen
Katalysator: $B_2O_3$-$Re_2O_7$/$Al_2O_3$-$SiO_2$, aktiviert mit einer Organoaluminiumverbindung

Der Versuch aus Beispiel 3 wird wiederholt. Dabei wird jedoch Methylaluminoxan durch eine Organoaluminiumverbindung gemäß Tabelle 1 ersetzt.

Bei einem molaren Verhältnis von $Re_2O_7$ : Al : 10-Undecensäuremethylester : 4-Octen = 1 : 4 : 80 : 160 werden die folgenden Undecensäure-Umsätze erzielt:

Tabelle 1

| Versuch | Aktivator | Umsatz |
|---|---|---|
| a | $Al(C_2H_5)_3$ | 84 % |
| b | $Al(i\text{-}C_4H_9)_3$ | 84 % |
| c | $C_2H_5Al(OC_2H_5)_2$ | 48 % |

Beispiel 8
Co-Metathese von 4-Octen mit 5-Decen
Katalysator: $B_2O_3$-$Re_2O_7$/$Al_2O_3$-$SiO_2$, aktiviert mit Methylaluminoxan

In einem mit $N_2$ gefüllten 50 ml fassenden zylindrischen Schlenkgefäß mit Magnetrührkern werden 1,08 g des in Beispiel 1 beschriebenen $B_2O_3$-$Re_2O_7$/$Al_2O_3$-$SiO_2$-Trägerkatalysators (0,098 mmol $Re_2O_7$) vorgelegt und mit 0,21 ml der im Beispiel 1 beschriebenen Methylaluminoxan-Lösung (0,392 mmol Al) sowie mit 23,5 ml einer äquimolaren Mischung von 4-Octen und 5-Decen versetzt, wodurch ein molares Verhältnis der einzelnen Komponenten wie folgt gegeben ist:
$Re_2O_7$ : Al : 4-Octen : 5-Decen = 1 : 4 : 800 : 800.

Die Mischung wird 2 Stunden bei Raumtemperatur gerührt. Der pulverförmige Trägerkatalysator wird absitzen gelassen. Von der überstehenden Lösung wird eine Probe entnommen, die nach Zugabe einiger Tropfen Methanol zur Zerstörung etwaiger Katalysatorreste gaschromatographisch analysiert wird. Gemäß der Reaktionsgleichung

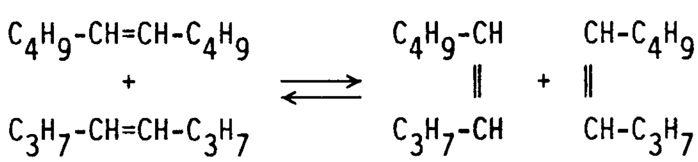

enthält die Reaktionsmischung neben den Einsatzverbindungen 4-Nonen. Der Umsatz der eingesetzten Olefine beträgt 50 %, die Selektivität zu 4-Nonen 97,6 %.

Beispiel 9
Co-Metathese von 4-Octen mit 5-Decen
Katalysator: $Re_2O_7$/$Al_2O_3$-$SiO_2$, aktiviert mit Methylaluminoxan

Der Versuch aus Beispiel 8 wird wiederholt, jedoch enthält der Rhenium-Trägerkatalysator jetzt kein $B_2O_3$. Außerdem werden die Katalysator- und die Aktivatorkonzentration verdoppelt.

Bei einem molaren Verhältnis von $Re_2O_7$ : Al : 4-Octen : 5-Decen = 1 :4 : 400 : 400 beträgt der Olefin-Umsatz 51 % bei einer Selektivität zu 4-Nonen von 96 %.

Beispiel 10
Homo-Metathese von 1-Octen
Katalysator: $B_2O_3$-$Re_2O_7$/$Al_2O_3$-$SiO_2$, aktiviert mit Methylaluminoxan

Der Versuch aus Beispiel 8 wird wiederholt, jedoch wird anstelle von 4-Octen/5-Decen nunmehr 1-Octen eingesetzt.

Bei einem molaren Verhältnis von $Re_2O_7$ : Al : 1-Octen = 1 : 4 : 1 600 beträgt der 1-Octen-Umsatz 86 %. Das Reaktionsprodukt ist eine Mischung aus über 10 Olefinen.

Beispiel 11
Homo-Metathese von 1-Octen
Katalysator: $Re_2O_7$/$Al_2O_3$-$SiO_2$, aktiviert mit Methylaluminoxan

Der Versuch aus Beispiel 10 wird wiederholt, jedoch enthält der Rhenium-Trägerkatalysator jetzt kein $B_2O_3$.

Bei einem molaren Verhältnis von $Re_2O_7$ : Al 1-Octen = 1 : 4 : 800 beträgt der 1-Octen-Umsatz 90 %. Die Reaktionsmischung ist ein Gemisch aus über 10 Olefinen.

Beispiel 12
Co-Metathese von 10-Undecensäuremethylester mit 4-Octen
Katalysator: $B_2O_3$-$Re_2O_7$/$Al_2O_3$-$SiO_2$, aktiviert mit Tetraethyldialuminoxan

Der Versuch aus Beispiel 3 wird wiederholt. Dabei wird jedoch Methylaluminoxan durch Tetraethyldialuminoxan (20%ig in Hexan, Al-Gehalt = 5,9 Gewichtsprozent) ersetzt.

Bei einem molaren Verhältnis von $Re_2O_7$ : Al : 10-Undecensäuremethylester : 4-Octen = 1 : 5,7 : 340 : 680 beträgt der 10-Undecensäuremethylester-Umsatz45 % bei einer Selektivität von 98 %.

Beispiel 13

Der Versuch aus Beispiel 12 wird wiederholt, wobei jedoch etwa die eineinhalbfache Mange an Katalysator und Aktivator eingesetzt wird. Dabei beträgt das molare Verhältnis von $Re_2O_7$ : Al : 10-Undecensäuremethylester : 4-Octen = 1 : 5,7 : 230 : 460. Der Umsatz des 10-Undecensäuremethylester steigt auf 77 % bei einer Selektivität von 87 %.

Beispiel 14
Co-Metathese von 10-Undecensäuremethylester mit 4-Octen
Katalysator: $B_2O_3$-$Re_2O_7$/$Al_2O_3$-$SiO_2$, aktiviert mit Tetraisobutyldialuminoxan

Der Versuch aus Beispiel 3 wird wiederholt. Dabei wird jedoch Methylaluminoxan durch Tetraisobutyldialuminoxan (20%ig in Heptan, Al-Gehalt = 3,6 Gewichtsprozent) ersetzt.

Bei einem molaren Verhältnis von $Re_2O_7$ : Al : 10-Undecensäuremethylester : 4-Octen = 1 : 5,7 : 340 : 680 beträgt der 10-Undecensäuremethylester-Umsatz39 % bei einer Selektivität von 98 %.

Beispiel 15

Der Versuch aus Beispiel 14 wird wiederholt, wobei jedoch etwa die eineinhalbfache Menge an Katalysator und Aktivitor eingesetzt wird. Dabei beträgt das molare Verhältnis von $Re_2O_7$ : Al : 10-Undecensäuremethylester : 4-Octen = 1 : 5,7 : 230 : 460. Der Umsatz des 10-Undecensäuremethylester steigt auf 56 % bei einer Selektivität von 94 %.

**Patentansprüche**

**1.** Verfahren zur Metathese von Olefinen und funktionalisierten Olefinen an $Re_2O_7$/$Al_2O_3$-haltigen Katalysatoren,
dadurch gekennzeichnet,
daß halogenfreie Organoaluminiumverbindungen als Aktivatoren zugesetzt werden.

**2.** Verfahren nach Anspruch 1,
dadurch gekennzeichnet,
daß Organoaluminiumverbindungen der Formel $X_{(3-m)}AlR_m$, wobei
R = Alkyl mit 1 bis 8 C-Atomen,
X = Alkoxy mit 1 bis 8 C-Atomen und
m = 1 bis 3 ist,
eingesetzt werden.

**3.** Verfahren nach Anspruch 1,
dadurch gekennzeichnet,
daß Alkylaluminoxane der Formel

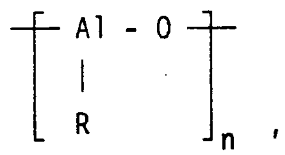

worin
R = Alkyl mit 1 bis 8 C-Atomen und
n = 2 bis 30 ist,
zugegeben werden.

**4.** Verfahren nach Anspruch 3,
dadurch gekennzeichnet,
daß Methylaluminoxane eingesetzt werden, in denen n eine Zahl von 5 bis 20 ist.

**5.** Verfahren nach Anspruch 1,
dadurch gekennzeichnet,
daß das molare Verhältnis $Re_2O_7$ : Aluminium in der Organoaluminiumverbindung bei 1 : 1 bis 1 : 10 liegt.

**6.** Verfahren nach Anspruch 1,
dadurch gekennzeichnet,
daß die funktionalisierten Olefine ungesättigte Carbonsäureester sind.

**Claims**

**1.** A process for the metathesis of olefins and functionalized olefins on $Re_2O_7/Al_2O_3$-containing catalysts, characterized in that halogen-free organoaluminium compounds are added as activators.

**2.** A process according to claim 1, characterized in that organoaluminium compounds of the formula $X_{(3-m)}AlR_m$ are used, in which
R = alkyl having from 1 to 8 C atoms,
X = alkoxy having from 1 to 8 C atoms and
m = 1 to 3.

**3.** A process according to claim 1, characterized in that alkylaluminoxanes are added of the formula

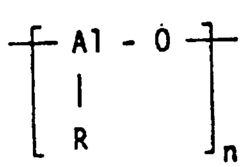

in which

R = alkyl having from 1 to 8 C atoms and
n = 2 to 30.

**4.** A process according to claim 3, characterized in that methylaluminoxanes are used in which n is a number from 5 to 20.

**5.** A process according to claim 1, characterized in that the molar ratio of $Re_2O_7$ : aluminium in the organoaluminium compound is from 1 : 1 to 1 : 10.

**6.** A process according to claim 1, characterized in that the functionalized olefins are unsaturated carboxylic esters.

**Revendications**

**1.** Procédé pour la métathèse d'oléfines et d'oléfines fonctionnalisées sur des catalyseurs renfermant un mélange de $R_2O_7$ et d'$Al_2O_3$,
caractérisé en ce que l'on ajoute en tant qu'activants des composés organo-aluminiques exempts d'halogène.

**2.** Procédé selon la revendication 1,
caractérisé en ce que l'on utilise des composés organoaluminiques de la formule

$$X_{(3-m)}AlR_m'$$

dans laquelle
R est un alkyle comportant de 1 à 8 atomes de carbone,
X est un alcoxy comportant de 1 à 8 atomes de carbone, et
m a une valeur de 1 à 3.

**3.** Procédé selon la revendication 1,
caractérisé en ce que l'on ajoute des alkyl-aluminoxanes de la formule

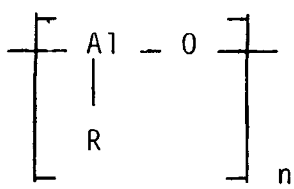
,

dans laquelle
R est un alkyle comportant de 1 à 8 atomes de carbone et
n a une valeur de 2 à 30.

**4.** Procédé selon la revendication 3,
caractérisé en ce que l'on utilise des méthyl-aluminoxanes, dans lesquels n est un nombre d'une valeur de 5 à 20.

**5.** Procédé selon la revendication 1,
caractérisé en ce que la proportion molaire entre $R_2O_7$ etl'aluminium dans le composé organo-aluminique se situe à une valeur de 1 : 1 à 1 : 10.

**6.** Procédé selon la revendication 1,
caractérisé en ce que les oléfines fonctionnalisées sont des esters insaturés d'acides carboxyliques.